# EUROPEAN PATENT APPLICATION

(11) **EP 2 284 161 A1**
(43) Date of publication of application: **16.02.2011**
(21) Application number: 09165998.7
(22) Date of filing: 21.07.2009
(51) Int. Cl.: C07D 277/82

(54) **Process for the preparation of riluzole**

(71) Applicant: EDMOND PHARMA S.R.L., 20157 Milano (IT)
(72) Inventor: Gatti, Pier Andrea, 20157, Milano (IT); Zacche', Matteo, 20157, Milano (IT); Gerli Fulvio, 20157, Milano (IT)
(74) Representative: Minoja, Fabrizio

(57) **Abstract**

A new and efficient process for the synthesis of Riluzole, a drug used to treat amyotrophic lateral sclerosis, is disclosed the process comprises the reaction 4-trifluoromethoxyaniline, a thiocyanate salt and an inorganic or organic oxidant in acetic acid solution.

## Description

### FIELD OF THE INVENTION

The present invention relates to a process for the synthesis of Riluzole.

### BACKGROUND OF THE INVENTION

2-Aminobenzothiazoles of formula **1** 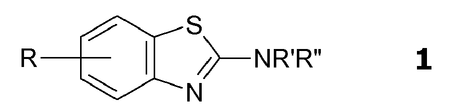 have been disclosed for the treatment of various central nervous system diseases. In particular, Riluzole of formula **2** is useful in the treatment of amyotrophic lateral sclerosis.

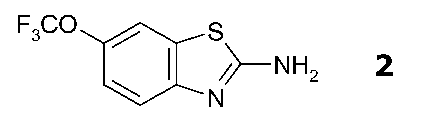

Moreover, US 4,370,338 discloses the use of Riluzole as having anticonvulsant, anxiolytic and hypnotic properties.

Kaufmann, Archiv. Pharm., 1928, 266, p. 197-218 describes the synthesis of compounds of formula **1** as a one-pot reaction between the appropriate aniline, potassium thiocyanate and bromine in acetic acid. Alkaline thiocyanate reacts with bromine in acetic acid to form thiocyanogen, which performs an electrophilic substitution on the aromatic ring and then a ring-closure reaction to form the benzothiazolic residue. This method provides only low to moderate yields, with the formation of many impurities due to the use of bromine. Moreover, many drawbacks are associated with the use of liquid bromine, because it is highly toxic to humans and to the ecological system, extremely corrosive, difficult to manipulate and emits toxic and unsafe fumes.

A similar method is provided in US 2008/0108827 which makes use of ammonium thiocyanate instead of potassium thiocyanate in the same reaction as before. This method suffers the same problems than the previous one regarding the use of bromine.

Another method is described by Hugerschoff, Chem. Ber., 1901, 34, p. 3130 and 1903, 36, p. 3121, who found that an arylthiourea can be cyclized with liquid bromine in chloroform to give the corresponding compound of formula 1. Although this reaction usually proceeds in good yields at low temperature, it suffers the same problems about bromine reagent. Moreover, this type of reaction makes use of chloroform which is toxic, carcinogenic and dangerous for the environment.

A modification of this method is provided in J. Org. Chem. 2003, 68, p. 8693-8696 which substitutes molecular bromine with an electrophilic bromine source, which is benzyltrimethylammonium tribromide. While this reagent is safer in respect to bromine, it is quite expensive and so it's not applicable to an industrial scale. Moreover, the desired product is obtained in moderate to low yields. Finally, the solvents required suffer by many ecological drawbacks.

There is thus a need for an efficient process for preparing Riluzole which uses eco-friendly solvents and reagents, which proceeds in high yields and which can be applied to an industrial scale.

### DESCRIPTION OF THE INVENTION

The present invention is based on the surprising discovery that Riluzole may be prepared by reaction between 4-trifluoromethoxyaniline and thiocyanogen reagent generated *in situ* by reaction between a thiocyanate salt and an inorganic or organic oxidant in acetic acid solution.

Suitable thiocyanate salts include but are not limited to sodium thiocyanate, potassium thiocyanate, ammonium thiocyanate, mercuric thiocyanate, lead thiocyanate or any other metal thiocyanate salt.

Suitable organic or inorganic oxidants include but are not limited to N-bromosuccinimide, hydrogen peroxide, sodium ipochlorite, sodium chlorite, sodium chlorate, sodium metaperiodate, sodium perchlorate, potassium permanganate, 2,3-dichloro-5,6-dicyanobenzoquinone (DDQ), potassium dichromate, potassium peroxide, potassium persulfate, potassium nitrosodisulfonate, pyridinium dichromate (PDC), pyridinium chlorochromate (PCC), Dess-Martin periodinane, sodium bromate, manganese dioxide, ammonium perchlorate, peracetic acid, potassium nitrate, sodium perborate, sodium percarbonate, sodium perphosphate, benzoyl peroxide, urea peroxide.

According to the present invention, the process is carried out by mixing 4-trifluoromethoxyaniline, the thiocyanate salt and the oxidant in acetic acid at a given temperature for the time required to complete the reaction. Water may be present also in the reaction medium.

The molar amount of the thiocyanate salt may be 1 to 10 moles per mole of the aniline, preferably 1 to 3 moles per mole.

The molar amount of the oxidant may be 1 to 10 moles per mole of the aniline, preferably 1 to 3 moles per mole.

Suitable temperatures range from 0°C to the boiling point of the solution, preferably from room temperature to 50°C.

The time required to accomplish the reaction may be as low as 30 minutes or frequently even 48 hours, depending on reaction conditions.

After reaction completion, raw Riluzole is separated from the reaction medium by methods known in the art. Suitable methods, for example, comprise neutralization of the acidic solution and extraction with a suitable solvent or filtration of the resulting solid, or liquid chromatography.

The raw product thus obtained is optionally submitted to a purification step which comprises for example recrystallisation from a suitable solvent or solvent mixtures or aqueous mixtures, by methods known in the art.

The process according to the present invention finally yields substantially pure Riluzole, with molar yields as high as 85% for the whole process, depending on reaction conditions.

Moreover, no bromine reagents and no toxic solvents are used, thus avoiding ecological and safety issues.

Finally most of the inorganic oxidants are of very common use and with very low costs.

In summary, the present invention discloses a simple, economical, efficient, robust, ecological friendly and suitable for industrial scale process for the manufacture of Riluzole in high yields and high purity. The process may be applied also to other compounds of general formula 1 as well.

Below are presented some examples which are provided for illustrative purposes only and therefore do not limit the scope of the invention itself in any way.

### EXAMPLE 1

4-Trifluoromethoxyaniline (10 g), potassium thiocyanate (13 g) and sodium bromate (8.5 g) are suspended in 50 mL of acetic acid and the suspension is stirred for 48 hours at 40°C. The resultant mixture is diluted with 200 mL of water and treated with ammonia until pH 14. The resulting solid is filtered and dried under vacuum to yield raw Riluzole. The raw product is then crystallized in a mixture of ethanol/water to yield substantially pure Riluzole in 46% molar yield.

### EXAMPLE 2

4-Trifluoromethoxyaniline (10 g), sodium thiocyanate (12 g) and manganese dioxide (5 g) are suspended in 50 mL of acetic acid and the suspension is stirred for 48 hours at room temperature. The resultant mixture is diluted with 200 mL of water, treated with sodium hydroxide until pH 14 and extracted with isopropyl ether. The organic layer is separated and concentrated under vacuum to yield raw Riluzole. The raw product is then crystallized in a mixture of ethanol/water to yield substantially pure Riluzole in 54% molar yield.

### EXAMPLE 3

4-Trifluoromethoxyaniline (10 g), ammonium thiocyanate (10 g) and DDQ (5.5 g) are suspended in 50 mL of acetic acid and the suspension is stirred for 48 hours at 45°C. The resultant mixture is diluted with 200 mL of water and treated with ammonia until pH 14. The resulting solid is filtered and dried under vacuum to yield raw Riluzole. The raw product is then purified by means of silica gel chromatography to yield substantially pure Riluzole in 63% molar yield.

### EXAMPLE 4

4-Trifluoromethoxyaniline (300 g), ammonium thiocyanate (300 g) and sodium metaperiodate (90 g) are suspended in 1.5 L of acetic acid and the suspension is stirred for 8 hours at 30°C. The resultant mixture is diluted with 1.5 L of water and treated first with 60 g of sodium bisulphite and then with ammonia until pH 14. The resulting suspension is extracted with isopropyl ether, the organic layer is then separated and concentrated under vacuum to yield raw Riluzole. The raw product is dissolved in 2.4 L of ethyl acetate and acidified with concentrated hydrochloric acid until pH 1. The resulting solid is filtered, dried under vacuum and dissolved in 1 L of ethanol, then treated with ammonia until pH 14. The resulting mixture is then diluted with water (1.4 L) and the resulting solid is filtered to yield substantially pure Riluzole in 75% molar yield.

### EXAMPLE 5

4-Trifluoromethoxyaniline (50 g), ammonium thiocyanate (65 g) and potassium persulfate (90 g) are suspended in 250 mL of acetic acid and the suspension is stirred for 24 hours at room temperature, then for 2 hours at 40°C to complete the reaction. The resultant mixture is diluted with 500 mL of water and 100 mL of ethanol, then treated with ammonia until pH 14. The resulting suspension is filtered and the solid is dried under vacuum to yield raw Riluzole. The raw product is then dissolved in a mixture of 150 mL of ethanol and 250 mL of water under reflux. The solution is then cooled to room temperature with crystallization of the desired product. The solid is then filtered and dried under vacuum to yield substantially pure Riluzole in 85% molar yield.

### EXAMPLE 6

4-Trifluoromethoxyaniline (50 g), ammonium thiocyanate (65 g) and potassium persulfate (90 g) are suspended in 250 mL of acetic acid and 25 mL of water; the exothermic reaction is stirred for 4 hours or until the temperature starts to decrease. The resultant mixture is diluted with 500 mL of water and 100 mL of ethanol, then treated with ammonia until pH 14. The resulting suspension is filtered and the solid is dissolved in ethyl acetate and neutralized with concentrated hydrochloric acid. The solution is concentrated under vacuum and then diluted with ethyl acetate causing the precipitation of the hydrochloride, which is filtered and dissolved in a mixture of 150 mL of ethanol and 250 mL of water and neutralized with ammonia. The solution is then cooled to 10°C with crystallization of the desired product. The solid is then filtered and dried under vacuum to yield substantially pure Riluzole in 81 % molar yield.

## Claims

1. A process for the preparation of Riluzole of formula 2 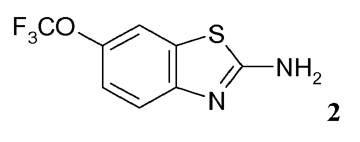 which process comprises reacting 4-trifluoromethoxyaniline with a thiocyanate salt and an oxidant in acetic acid medium, optionally with the presence of water.

2. The process of claim 1 wherein the thiocyanate salt is selected from sodium thiocyanate, potassium thiocyanate and ammonium thiocyanate.

3. A process according to claim 1 wherein the oxidant is selected from sodium bromate, manganese dioxide, 2,3-dichloro-5,6-dicyanobenzoquinone (DDQ), sodium metaperiodate and potassium persulfate.

4. A process according to claim 1 wherein the molar amounts of the thiocyanate salt and of the oxidant are 1 to 10 moles per mole of 4-trifluoromethoxyaniline.

5. The processa according to claim 4 wherein the molar amounts of the thiocyanate salt and of the oxidant are 1 to 3 moles per mole of 4-trifluoromethoxyaniline.

6. A process according to claim 1 wherein the reaction is performed at a temperature ranging from room temperature to 50°C.
